# EUROPÄISCHE PATENTSCHRIFT

(11) **EP 1 721 949 B1**
(45) Veröffentlichungstag und Bekanntmachung des Hinweises auf die Patenterteilung: **16.06.2010**
(21) Anmeldenummer: 06009182.4
(22) Anmeldetag: 04.05.2006
(51) Int. Cl.: C09J 4/00

(54) **Haftverbessernde Additive für polymerisierbare Zusammensetzungen**
Adhesive with improved adhesive strength for polymerisable composition
Adhésive pour une adhérence améliorée pour composition polymérisable

(30) Priorität: 13.05.2005 DE 102005022172
(43) Veröffentlichungstag der Anmeldung: 15.11.2006
(73) Patentinhaber: VOCO GmbH, 27472 Cuxhaven (DE)
(72) Erfinder: Krumme, Wigand Florenz, 27472 Cuxhaven (DE); Gerkensmeier, Thorsten, 27472 Cuxhaven (DE); Plaumann, Manfred Thomas, 27472 Cuxhaven (DE)

(56) Entgegenhaltungen:
- EP-A- 0 854 154
- US-B1- 6 245 828

## Beschreibung

### Stand der Technik

Über einen weiten Zeitraum hinweg wurde die zahnärztliche Füllungstherapie durch den Einsatz von Amalgam bestimmt. Da Amalgam keine eigene Haftung an Zahnhartsubstanz aufweist, muss der Füllungswerkstoff mechanisch, beispielsweise durch die Präparation von Unterschnitten oder Verkeilungen bei der definitiven Versorgung von Kavitäten zum Halten und Verbleib in der Kavität gebracht werden. Ein solches Verfahren verbraucht jedoch gesunde Zahnhartsubstanz im Übermaß und wird dem heutigen Standard der restaurativen Zahnheilkunde mit der Forderung nach minimalinvasiven Präparationen und möglichst substanzschonenden Kavitätendesign nicht mehr gerecht. Weil Amalgam keine Eigenhaftung zur Zahnhartsubstanz zeigt, tritt beim Einsatz von Amalgam stets ein Randspalt zwischen der Präparation und dem Zahnfüllmaterial auf. Dieser Randspalt wird in den der Füllungslegung folgenden Wochen durch bakterizide Korrosionsprodukte des Amalgams und sonstigen Ablagerungen weitgehend aufgefüllt und ein Eindringen von kariogenen Keimen wirksam verhindert. Je größer der Randspalt ausfällt, desto geringer ist der Anteil an Amalgamkorrosionsprodukten im Spalt und ein Eindringen von Bakterien wird immer wahrscheinlicher. Ein breiter Randspalt führt zu einer raschen Besiedlung mit Mikroorganismen, die zur Ausbildung einer Sekundärkaries und damit dem Füllungsverlust führen können. Zusätzlich gilt das Amalgam toxikologisch als bedenklich und ist zudem vom ästhetischen Gesichtspunkt aus völlig unbefriedigend.
Dies sind die Gründe, warum ein stetig wachsender Anteil der Patienten zahnfarbene Kunststoffmaterialien oder andere Alternativen dem Amalgam zur Versorgung ihrer Kavitäten vorziehen.
Mit dem Aufkommen von härtbaren, plastischen Kunststoffen als alternative Füllungsmaterialien in der konservierenden Zahnheilkunde ergaben sich neue verschiedene Problemfelder, die bis heute noch nicht umfassend zufrieden stellend gelöst sind. Zu den Schwachpunkten plastischer Füllungskunststoffe zählen außer dem erhöhten Abrieb und der biologischen Verträglichkeit der so genannte Polymerisationsschrumpf und das Problem des dauerhaften, randspaltfreien Verbundes mit der Zahnhartsubstanz, welches die Haltbarkeit der Restauration häufig gefährdet.

Werden Kunststoffe ausgehärtet, so findet beim Übergang von der flüssigen zu der festen Phase eine Änderung der Dichte statt. Dieses Phänomen, auch Schrumpf genannt, kann dazu führen, dass eine dauerhafte und feste Verbindung zwischen der Zahnsubstanz und dem Füllungsmaterial nicht aufgebaut werden kann, da durch die Polymerisationsschrumpfung hohe Zugkräfte auf den Kunststoff-Zahnsubstanz-Verbund wirken. Dieser Effekt kann außerdem durch zeitlich versetzte, gegenläufige Quellungseffekte durch Aufnahme oraler Flüssigkeiten weiter kompliziert werden. Die Aufnahme von Flüssigkeiten aus dem Mundraum wird in erster Linie durch die Polarität der Kunststoffkomponenten der Füllung und des Adhäsivs bestimmt. Hoch polare Materialien neigen zur Aufnahme größerer Mengen Wasser aus dem Speichel und führen zu einer Volumenzunahme sowie zur allmählichen Ablösung des Kunststoffes von der Zahnhartsubstanz. Die aufgenommene Feuchtigkeit kann darüber hinaus Hydrolyseprozesse in Gang setzen, die häufig organische oder anorganisch-organische Ester betreffen und zu einer beträchtlichen Schwächung des Haftverbundes und der physikalischen Eigenschaften der Füllung führen können, die sich dann nachteilig auf die Langzeitprognosen der Haltbarkeit auswirken. Besitzt das Füllungsmaterial keine ausreichende Haftkraft am Kavitätenrand, so bilden sich feine Randspalte zwischen dem Zahn und der Restauration, die häufig für Hypersensibilitäten verantwortlich sind und ein Eindringen von Flüssigkeiten und Bakterien an der Dentin-Kunstoffgrenze entlang der Kavitätenwand in die Tiefe ermöglichen. Eine solche unzureichende Randadaption des dentalen Füllungskomposits kann demnach eine bakterielle Unterminierung der Restauration mit nachfolgender Sekundärkariesbildung und schwerer Schädigung des Zahnes verursachen. Die Folgen können von einer Randverfärbung über eine pulpitische Reizung bis hin zur Zahnmark- und Zahnwurzelvereiterung reichen, die letztendlich zum Verlust der Restauration und gegebenenfalls des Zahnes führen.
Fortschritte im Verfahren der adhäsiven Bindung von Füllungsmaterialien an das harte Zahngewebe erfolgten in der Vergangenheit nur in kleinen Schritten und sind in der Literatur beschrieben.
Ein Grund für den unzureichenden Verbund zwischen Zahnsubstanz und Kunststoff liegt in der Struktur des Dentins begründet, die als Folge eines zur Mundhöhle gerichteten osmotischen Drucks des Dentinliquors in den Dentintubuli immer eine gewisse Feuchtigkeit aufweist. Darüber hinaus besteht das Dentin zu einem großen Teil organischer Substanzen, insbesondere Kollagen, in dem die anorganischen Hydroxylapatitkristalle eingelagert sind. Dieser Strukturtyp ist sehr viel komplizierter aufgebaut als der Zahnschmelz. Zudem entsteht bei der Präparation am Dentin auf der Oberfläche eine Schmierschicht, die aus Zahnhartsubstanzbestandteilen, Bakterien, Speichel und Blut besteht und weder mechanisch noch durch Spülung zu entfernen ist.
Diese Voraussetzungen erschweren den Aufbau eines beständigen Verbundes zwischen Zahnsubstanz und Kunststoff in ganz erheblichem Maße.
Eine erste Überlegung führte zur Verwendung oberflächenaktiver Monomere als Verbindungsmittel zwischen der hydrophilen Zahnsubstanz und dem hydrophoben Füllungskunststoff. Bowen schlug das Additionsprodukt zwischen der Aminosäure N-Phenylglycin und Glycidylmethacrylat (NPG-GMA) als Mittel zur Verbesserung der adhäsiven Bindung vor (US 3200142). Die eine endständige Carbonsäurefunktion von N-(2-hydroxy-3-methacryloxypropyl)-N-phenylglycin sollte als funktionelle Gruppe eine Bindung zu den Kalziumionen des im anorganischen Bestandteil des Dentins enthaltenen Hydroxylapatits aufbauen, während die ethylenische Doppelbindung der Methacrylatgruppe eine kovalente Bindung zum Füllungskunststoff während der Polymerisation sicherstellen sollte.
In ähnlichen Ansätzen wurden monomere Bindungsagentien eingesetzt, die außer der Carbonsäurefunktion (oder einer zu einer Cabonsäurefunktion überführbaren Gruppe) auch andere oberflächenaktive Struktureinheiten wie Phosphat-, Sulfonat-, Sulfinat-, Isocyanat-, Hydroxyl- und Amidgruppen enthalten konnten. Ein alternativer Ansatz zum Aufbau eines randspaltfreien Verbundes zwischen Füllungskunststoff und der Zahnhartsubstanz bestand darin, Haftsysteme zu formulieren, die sich mit den organischen Bestandteilen des Dentins, dem Kollagen, und einer endständigen Gruppe des Haftvermittlers umsetzen konnten. Eingesetzt wurden hier beispielsweise Aldehydgruppen aufweisende Verbindungen wie Glutaraldehyd (EP 0 141 324). Die Aldehydfunktion sollte beispielsweise mit einer Aminofunktion des Proteins im Kollagen in einem ersten Schritt zu einem Aminoalkohol umgesetzt werden, um dann im zweiten Schritt unter Abspaltung von Wasser zu einer Schiffschen Base abzureagieren. Es wurde weiterhin vorgeschlagen, die aldehydgruppenhaltigen Verbindungen zusammen mit aktiven Wasserstoffatomen ausgestatteten Monomeren, wie beispielsweise Hydroxyethylmethacrylat, einzusetzen, um sicherzustellen, daß die Eliminierung von Wasser und somit die Umsetzung der zweiten Reaktionsstufe und Kopplung des Haftmonomers an das Dentin tatsächlich stattfindet, weil Schiffsche Basen unter den wässrigen Bedingungen der Mundhöhle möglicherweise nicht stabil genug sind. Ferner wurde versucht, zusätzlich zur Aldehydreaktion eine gezielte Pfropfung des Kollagens durch mit Tri-n-butylboran initiierte Pfropfcopolymerisation von Methacrylsäureestern durchzuführen (US 48,30,616).
Eine weitere Verfeinerung des Systems beschreibt die DE 41 37 076. Anstelle der aldehydgruppenaufweisenden Verbindungen wurden hier β-Dicarbonylverbindungen, wie beispielsweise 2-Acetacetoxyethylmethacrylat, eingesetzt. Es wurde angenommen, daß die β-Carbonylfunktion eine erheblich größere Reaktivität gegenüber dem Protein, d.h. dem Kollagen im Vergleich zur Aldehydgruppe aufweist und zusätzlich die bekannt guten Komplexierungseigenschaften der β-Carbonylgruppe eine Rolle spielen.
Weiterhin wurde versucht, durch eine Kombination der Strategien Adhäsivzusammensetzungen bereitzustellen, die sowohl an Kollagen als auch an Kalzium haften (EP 0 321 683).
In der Praxis zeigte sich jedoch, daß die Haftwerte aller oben genannten Systeme nach kurzer Zeit stark nachließen. Im weiteren Verlauf des Bemühens, geeignete und zuverlässige Haftmonomere zu entwickeln, konnten schließlich einige wenige und ganz spezielle Verbindungen aufgefunden werden, die eine erhöhte Haftwirkung zwischen der Zahnsubstanz und dem Füllungsmaterial auch nach Alterung der Systeme aufwiesen. Diese Verbindungen wurden dann auch in Dentalmaterialien eingesetzt und kommerziell vertrieben. Diese aktuellen Haftmittel erfordern verschiedene Verarbeitungsmethoden, um erfolgreich mit Zahnhartsubstanzen verbunden zu werden. Allen gemeinsam ist die Veränderung des Schmelzes oder Dentins durch Säuren, Primer oder Conditioner, deren Aufgabe es vereinfacht gesprochen ist, die Oberfläche durch Schaffung retentiver Muster aufzurauhen. Diese Ätzung erfolgt zumeist in einem separaten Schritt. Flüssige unpolare Harzmischungen können dann, häufig vermittelt durch polare, flüchtige Lösungsmittel, in die retentiven Oberflächen einziehen und gehärtet werden. Die verwendeten Säuren, Primer und Conditioner enthalten häufig organische oder anorganische Säuren wie beispielsweise Phosphorsäure oder Zitronensäure, die aufgrund ihres niedrigen pH-Wertes die anorganischen Bestandteile über einen bestimmten Zeitraum auflösen und dann entfernt werden müssen. In anderen modernen Bond-Materialien wird die Ätzung des Haftgrundes mit dem Auftragen des Adhäsivs durch die Verwendung haftungsfördernder, saurer und polymerisierbarer Verbindungen kombiniert. Als eine Haftung vermittelnde Verbindung aus der Gruppe der Mono- oder Diphosphatester der Hydroxyalkylmethacrylate erwies sich 10-Methacryloyloxydecyldihydrogenphosphat (10-MDP) (EP 0 074 708, EP 1 084 131). Die Phosphorsäurefunktion bildet mit dem Hydroxylapatit stabile, wasserunlösliche Salze, wobei das Kalzium mit Hilfe der Phosphorsäuregruppe komplexiert wird. Ganz offensichtlich spielt die Anzahl der zehn Methylengruppen als Abstandshalter zwischen der reaktiven Phosphorsäure- und der Methacrylatgruppe eine entscheidende Bedeutung bei der Wirksamkeit dieses Haftmonomers. Der Methylenspacer scheint eine genau abgestimmte Länge aufzuweisen, um gegenseitige Störungen durch sterische Effekte bei der Bindungsbildung an beiden Enden des Haftvermittlers zu vermeiden.
Dies wiederum scheint eine Voraussetzung zu sein, um die Substratoberfläche optimal und gleichmäßig benetzen zu können.
Präparativ wird 10-MDP durch Reaktion von 10-Hydroxydecylmethacrylat mit Phosphoroxychlorid erhalten.
Andere, Haftung vermittelnde Verbindungen sind Methacryloyloxyalkylderivate aromatischer Carbonsäuren. Es zeigte sich, daß insbesondere Trimellitsäure-4-methacryloyloxyethylester (4-MET) oder Trimellitsäureanhydrid-4-methacryloyloxyethylester (4-META) als haftungsfördernde Monomere eingesetzt werden können (DE 28 28 381, US 4 148 988, EP 0 684 033, EP 0 684 034). 4-META kann durch Dehydrochlorierungsreaktion zwischen Hydroxyethylmethacrylat und wasserfreiem Trimellitsäurechlorid oder durch Dehydrationsreaktion zwischen Hydroxyethylmethacrylat und Trimellitsäureanhydrid hergestellt werden.
Weitere reaktive Haftkomponenten werden in EP 1 148 060, EP 0 909 761 und EP 1 148 071 offenbart, wo polymerisierbare und hydrolysestabile Acrylphosphonsäuren beschrieben sind. Der aufwendige Syntheseweg beginnt mit der Umsetzung von Formaldehyd und einem geeigneten Acrylsäureester in Gegenwart eines Katalysators unter Bildung einer Methylolgruppe in α-Position des Esters und anschließender Halogenierung des Alkohols mit einem anorganischen Säurehalogenid. Der so hergestellte α-Halogenmethacrylsäueester wird dann mit geeigneten und zuvor geschützten mono- oder difunktionellen Phosphonsäureestern umgesetzt. Nach Abspaltung der Schutzgruppe wird dann die polymerisierbare und hydrolysestabile Acrylphosphonsäure erhalten, deren Merkmal die endständige Oxo-ethylacrylatfunktion darstellt. Anhand von Vergleichsbeispielen mit herkömmlichen Acrylphosphonsäuren konnten die Erfinder zeigen, daß eine Esterhydrolyse bei den neuen Phosphonsäuren dann nicht mehr zu einer Trennung von Methacrylat- und Phosphonsäurestruktur im Haftmonomer führt und auf diesem Weg eine recht permanente Bindung zwischen Füllungskunststoff und Zahnstruktur gewährleistet ist.
Ein weiterer Typ eines Haftmonomers ist der Phosphorsäureester von Dipentaerythritolpentaacrylat (PENTA, US 4,514,342). Der Ester wird aus Dipentaerythritolmonohydroxypentaacrylat und Phosphoroxychlorid in Gegenwart von Triethylamin hergestellt.
In der EP 1 346 717 wird Tetramethacryloxyethylpyrophosphat als Haftung vermittelnde Substanz beschrieben. Das Pyrophosphat bricht unter den wässrigen Bedingungen auf und hydrolysiert zu Phosphorsäureresten, die anfänglich für einen sehr niedrigen pH-Wert sorgen und helfen, das Hydroxylapatit anzuätzen. Die Phosphorsäurereste werden dann durch Kalziumionen, die aus dem Dentin herauswandern, neutralisiert und bilden so einen zementartigen Haftverbund am Zahn, während die Methacrylatgruppen durch Lichtpolymerisation mit dem Zahnfüllungsmaterial abreagieren können.
Die genannten Haftvermittler sind sehr aufwendig herzustellen, ihre Haftstärke an Schmelz und Dentin sind oft sehr unterschiedlich und stark abhängig von der Art der Anwendung. Weiterhin erwiesen sich auf Phosphor- und/oder Sulfonsäurefunktionen basierende Haftmonomere aufgrund des sehr niedrigen pH-Wertes als wenig biokompatibel im Hinblick auf das permeable Dentin. Postoperative Sensibilitäten und pulpitische Beschwerden infolge des Einsatzes solcher Verbindungen wurden in der Literatur häufig berichtet.

### Aufgabe der Erfindung

Es ist daher die Aufgabe der vorliegenden Erfindung, einen einfach herzustellenden Haftvermittler zur Verfügung zu stellen, der gegenüber Dentin und Schmelz eine Haftkraft von wenigstens 10 MPa aufweist, leicht zu verarbeiten ist und von hoher Biokompatibilität und Beständigkeit ist.

### Beschreibung der Erfindung

Gelöst wird diese Aufgabe durch den Einsatz substituierter Alkylendiamintetraessigsäurederivate der Formel 5, wobei R¹ und R² für Ethylen stehen. Ethylendiamintetraessigsäure (EDTA) weist eine geringe Toxizität auf und ist chemisch stabil, kann jedoch aufgrund seiner Funktionalitäten mit einer Vielzahl verschiedener organischer und anorganischer Substanzen chemisch verbunden werden. Es konnte gezeigt werden, daß die Verknüpfung des komplexbildenden EDTAs mit einem organischen, polymerisierbaren Molekül interessante, dauerhaft haftverbessernde Eigenschaften von Adhäsiven herbeiführen kann. Ethylendiamintetraessigsäure (EDTA) ist bekannterweise in der Lage, eine Reihe von Metallionen zu komplexieren. Diese Eigenschaft wird in der Komplexometrie zur naßchemischen Bestimmung verschiedener Metallionen genutzt. Die besondere Affinität des Dinatriumsalzes der Tetraessigsäure bezüglich des Kalziumions wurde in der Vergangenheit häufig zur Wasserenthärtung genutzt, um beispielsweise die Wirksamkeit von Tensiden zu optimieren. Ohne Anspruch darauf zu erheben, daß dieses Modell der Wahrheit entspricht, wird die besondere komplexbildende Eigenschaft des Ethylendiamintetraessigsäurederivates in der Literatur auf die Fähigkeit zur Ausbildung eines oktaedrischen Ligandenfeldes zurückgeführt (siehe Abbildung 1). Stickstoff- und Sauerstoffatome des Moleküls nehmen die Eckpunkte eines Oktaeders ein. Die Kanten des Oktaeders in der Abbildung bezeichnen keine Bindungen, sondern helfen bei der Orientierung. Die Verknüpfungen im Molekül werden durch die grauen Bögen gekennzeichnet. Ob ähnliche Strukturen auch in den erfindungsgemäßen Beispielen gebildet werden, ist nicht geklärt, allerdings helfen diese Betrachtungen, eine Vorstellung des möglichen Wirkmechanismus zu gewinnen.

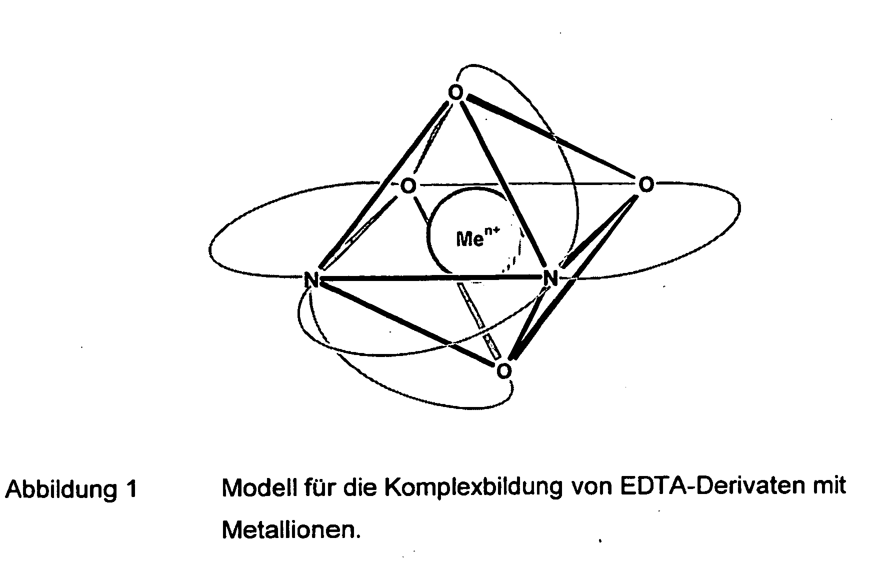

EDTA Komplexe mit Salzen können als ungelöste oder gelöste Metallkomplexe vorliegen. Um sie zu immobilisieren und in einen Haftverbund mit einer organischen Matrix zu integrieren, muß das Molekül chemisch modifiziert werden. Eine solche chemische Modifikation ist die kovalente Verbindung des EDTA mit ethylenisch ungesättigten Monomeren wie beispielsweise Acryl- oder Methacrylsäurederivaten, und Styrolen und auch mit Oxiranen. Darüber hinaus sind zahlreiche weitere chemische Funktionalitäten bekannt, die zur Polymerisation mit dem EDTA verknüpft werden können und somit dem Grundgedanken dieser Anmeldung entsprechen wie beispielsweise Di- oder Polycarbonsäuren oder deren Derivate sowie Polyole und Polyamine als Bausteine für Polykondensationsreaktionen.
Die Synthese von Alkylendiamintetraessigsäuren erfolgt nach Standardverfahren. Typischerweise werden in einem ersten Schritt die Diamine mit α-Halogenessigsäurederivaten unter Bedingungen umgesetzt, die die Bildung eines alkylierten Amins begünstigen und die Amidbildung verhindern. Reaktionen dieser Art sind in der Literatur beschrieben und lassen sich in guten Ausbeuten durchführen. Von allen auf diese Weise zugänglichen Diaminotetraessigsäuren ist das Produkt aus der Umsetzung mit 1,2-Diaminoethan, das EDTA, industriell am bedeutsamsten.

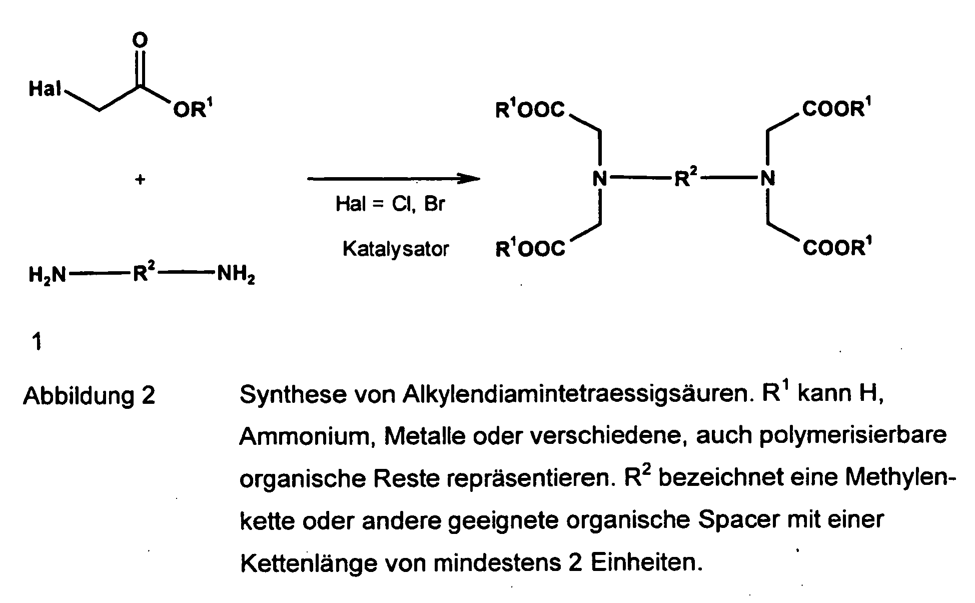

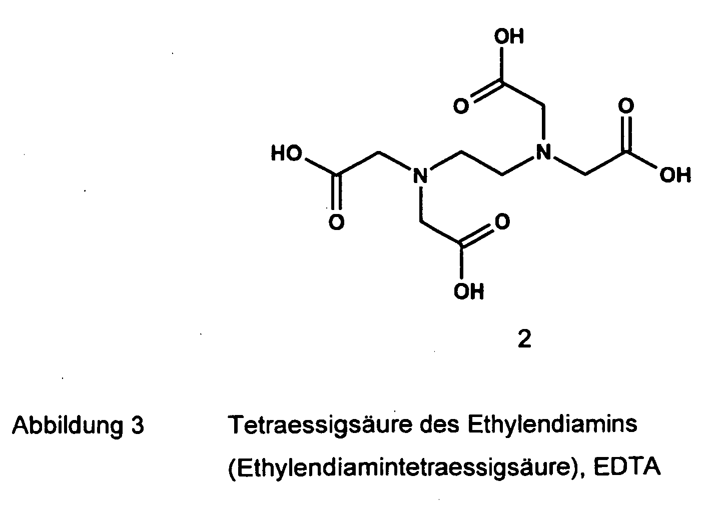

EDTA 2 ist ein in Wasser schlecht und den meisten organischen Lösungsmitteln wenig lösliches, organisches Material. Erst als Salz oder durch Verknüpfung mit anderen organischen Molekülen lassen sich gut lösliche Verbindungen erhalten. Durch die Auswahl eines geeigneten organischen Substituenten läßt sich sowohl die Löslichkeit verbessern, als auch die Fähigkeit zur Anbindung an ein Polymer in einem Schritt in das Molekül einbringen. Ein Beispiel für die Verbesserung der Löslichkeit und gleichzeitigem Einbringen einer polymerisierbaren Gruppe ist das Veresterungsprodukt von EDTA und Hydroxyethylmethacrylat (HEMA). Dieses Produkt vereinigt die Eigenschaften, Calciumionen zu komplexieren und radikalisch polymerisierbar zu sein. Die Synthese der gewünschten Verbindung läßt sich in guten Ausbeuten und hoher Reinheit aus EDTA-Dianhydrid und HEMA realisieren. EDTA-Dianhydrid wird nach bekannten Vorschriften aus der freien Säure durch Erhitzen in Acetanhydrid erhalten. Als Katalysator wirken Triethylamin, Pyridin oder N,N-Dimethylaminopyridin (Patent, F. Budsky, J. Prokop, P. Raban, J. Seblova, CS 272584).

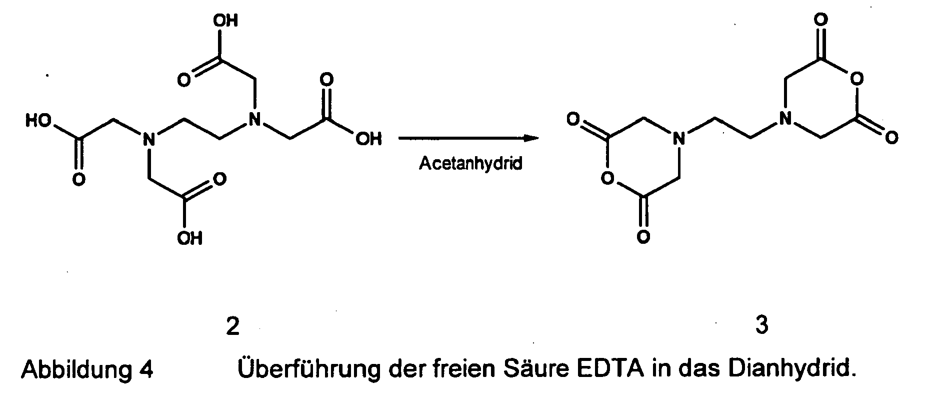

EDTA 2 reagiert in Acetanhydrid in guten Ausbeuten zum EDTA-Dianhydrid 3. EDTA-Dianhydrid ist ein farbloses, feines Pulver, das gut verschlossen und vor Feuchtigkeit geschützt aufbewahrt werden sollte. Wie die meisten anderen Carbonsäureanhydride reagiert es empfindlich auf Feuchtigkeitsspuren und Amine sowie andere basisch wirkende Chemikalien. Es ist gut löslich in Aceton, wenig löslich in Dichlormethan und unlöslich in Diethylether und Cyclohexan. Das sorgfältig gewaschene und getrocknete Produkt kann direkt für die nächste Umsetzung eingesetzt werden.

Die Umsetzung von 3 mit dem Alkohol Hydroxyethylmethacrylat (HEMA) wird durch die Möglichkeit zur Bildung von Mono- und Diestern kompliziert. Am Einfachsten erscheint die Reaktion eines Äquivalentes des Dianhydrides mit mindestens zwei Äquivalenten des Alkohols, um eine vollständige Umsetzung des reaktiven Anhydrids zum Diester 5 zu gewährleisten. Die Bildung des Monoesters 4 wird auf diese Weise weitgehend vermieden.

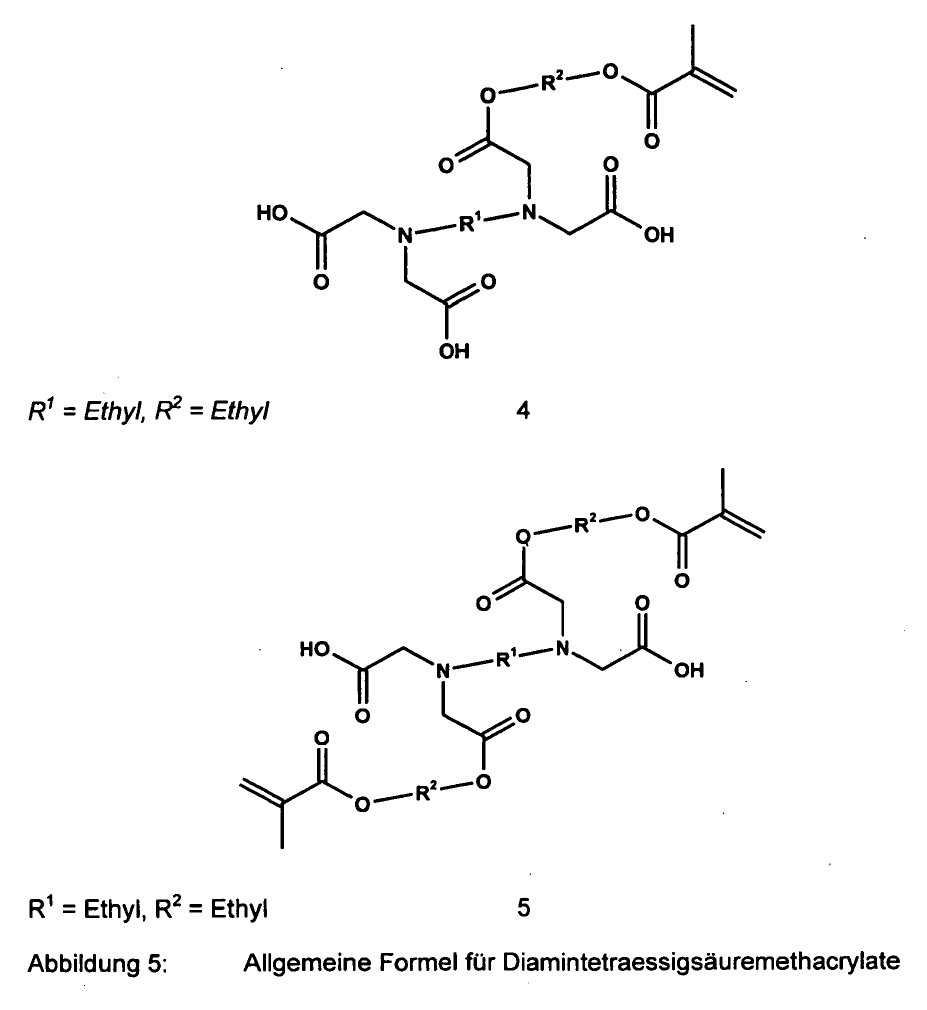

### Adhäsive Mischungen

Die adhäsive Verbindung von Kompositen mit Zahnhartmaterialien wird in der Regel durch die Verwendung des "Total-Etch" Verfahrens oder Primern erreicht, die die Zahnoberflächen für die Aufnahme von Adhäsivharzen vorbereiten. Die beim "Total-Etch" Verfahren eingesetzte, konzentrierte Phosphorsäure soll durch partielle Auflösung von Schmelz und Dentin und Öffnung der Dentinporen das Einfließen des Haftadhäsivs und die retentive Verankerung sicherstellen. Diese Dentinätzung mit Phosphorsäure ist ein zusätzlicher Arbeitsschritt bei der restaurativen Füllungstherapie, der sowohl aufwendig als auch relativ fehleranfällig ist. Zu langes wie auch zu kurzzeitiges Einwirken lassen des Ätzmittels führt zu Problemen beim Haftverbund und unvollständige Entfernung bedingt häufig langandauernde pulpitische Beschwerden und Hypersensibilität. Andere Haftsysteme benötigen Primer, die die Kavitätenoberflächen modifizieren und vor der Anwendung des eigentlichen Adhäsivharzes zeitaufwendig verarbeitet werden müssen. Neuere Dentaladhäsive arbeiten ohne Total-Etch und sind selbstkonditionierend. Diese Präparate sind wesentlich einfacher, anwendungssicherer und sparen bei der Behandlung Zeit. Für die vorliegenden Beispiele wurden daher Adhäsive erstellt, die nach dem Non-Etch Verfahren und ohne Primer verarbeitet werden.

Verbindungen des Typs 5 sind als Additiv für haftverbesserte Adhäsive speziell für mineralische Untergründe und besonders Zahnhartmaterialien geeignet. Sie erlauben die Anwendung im Non-Etch Verfahren, da die erfindungsgemäßen Substanzen als Bestandteil der Harzmischung offenbar die für das Bondingprozedere notwendige Dentinmodifizierung bewirken. Die Reinverbindung 5 ist ungeeignet, einen permanenten sicheren Haftverbund zu gewährleisten. Wie bereits bei den handelsüblichen Monomerharzen bekannt, sind die Polymere eines einzigen Harzmonomers bezüglich der physikalischen Eigenschaften minderwertig und geeigneten Harzmischungen häufig unterlegen. Um dieses Problem zu umgehen, werden Harzmatrices aus verschiedenen reaktiven, vernetzbaren Dimethacrylaten sowie Monomethacrylaten mit gebrauchsüblichen Anteilen an Initiatoren für die thermisch-, licht- oder redoxinduzierte Härtung hergestellt und mit Haftvermittler 5 versetzt. Harzkomponenten für die Zusammenstellung eines adhäsiven Gemisches können verschiedene, übliche Substanzen, zum Beispiel HEMA, Urethandimethacrylate (UDMA), Bisphenol-A-glycidyldimethacrylat (Bis-GMA), Polyethylenglycoldimethacrylate (TEDMA, TEGDMA), Trimethylolpropantrimethacrylat (TMPTMA) oder Dodecandioldimethacrylat (DODMA) beinhalten. Übliche Initiatoren für die Lichthärtung mit blauem Licht sind beispielsweise Campherchinon und N,N-Dimethylaminobenzoesäureethylester (DABE), Phospinoxide und Aryliodoniumsalze. Geeignete Initiatoren für die chemische radikalische Härtung sind unter anderem Peroxide und Amine oder Sulfinsäuren/-säuresalzen wie beispielsweise Dibenzoylperoxid (BPO) in Verbindung mit N,N-bis(hydroxyethyl)paratoluidin (N,N-Bis) oder Natriumsulfinat sowie Barbitursäurederivate wie Benzylphenylbarbitursäure neben löslichen Kupfer- und Tetraalkylammoniumchloridsalzen, Trialkylborane und Azobisisobutyronitril (AIBN). Als Schutz vor ungewünschter, vorzeitiger Polymerisation der Methacrylat- oder Acrylatmischung werden Stabilisatoren in gängigen Gehalten eingesetzt. Beispiele hierfür sind Butylhydroxytoluol (BHT) und Hydrochinonmonomethylether (HQME). Diese Stabilisatoren können auch zur Regelung der Redoxinitiierung eingesetzt werden. Zur Verbesserung der Materialeigenschaften können Feinpartikelfüllstoffe aus der Gruppe der Kieselsäuren, Glaskeramiken, Glasionomere, Aluminiumoxid, Quarz, Apatit oder anderen kristallinen oder amorphen Mineralstoffen oder Solen unterschiedlicher Partikelgrößen eingesetzt werden. Auch organische Füllstoffe wie zum Beispiel aufgemahlene Polymere und Präpolymere unterschiedlicher Körnung sind grundsätzlich anwendbar.

Für die genannten Beispiele wurden ausschließlich einkomponentige, aktivierte Harzmischungen eingesetzt, d.h. die Adhäsive enthalten keine Redoxkatalysatoren und werden in diesem Fall durch Lichtinitiierung polymerisiert. Zur Überprüfung der Haftung werden Haftserien im "Self-Etch" Verfahren angefertigt, d.h. das Adhäsiv wird nach der Kavitätenpräparation, -reinigung und Trocknung direkt auf das Dentin aufgetragen und eingearbeitet. Als Substrat dienen Dentin- und Schmelzproben von frischen extrahierten menschlichen Molaren, die nach gründlicher Reinigung und Desinfektion in Methylmethacrylat (MMA) eingegossen und nach der Polymerisation in Scheiben gesägt wurden. Diese Probenkörper werden dann soweit beschliffen, daß eine plane Dentin- oder Schmelzfläche entsteht. Bis zu ihrer Verwendung werden diese Proben dann in isotonischer Kochsalzlösung aufbewahrt. Es ist wesentlich, daß die Oberflächen zu keinem Zeitpunkt vollständig austrocknen, da sich hierdurch die Fähigkeit des Dentins, Haftadhäsive aufzunehmen, irreversibel verringert und der Haftverbund gefährdet wird. Für den Haftprozeß werden die Proben zunächst unter fließendem Wasser abgespült und überschüssiges Wasser mit dem Luftbläser vertrieben, so daß eine matt glänzende, aber immer noch feuchte Oberfläche bleibt. Danach wird das Adhäsiv mittels eines Pinsels oder Schwämmchens in die Dentinoberfläche mindestens 15 Sekunden eingearbeitet, Lösungsmittel und Überschüsse verblasen und 20 Sekunden lichtgehärtet. Anschließend wird auf der gehärteten Adhäsivoberfläche ein zylindrischer Silikonring mit einem Innendurchmesser von ca. 5 mm platziert und ein Füllungskomposit eingebracht. Nach dem Lichthärten des Füllungskomposits wird ein Teil der Proben für 1 Stunde und ein anderer Teil der Proben für 24 Stunden im Wasserdampfbad bei 37°C gelagert, dann der Silikonring entfernt und die Scherhaftfestigkeit ermittelt.

Da die Haftwerte von dentalen Bondingsystemen sehr vielen Einflüssen unterworfen sind, sind Einzeluntersuchungen von geringem Wert. Aus diesem Grunde wurde die haftvermittelnde Wirkung der erfindungsgemäßen Verbindungen durch den direkten Vergleich im Wesentlichen zusammensetzungsgleicher Adhäsivmischungen entweder mit 4-META oder 5 untersucht. 4-META ist ein für seine guten, haftvermittelnden Eigenschaften bekanntes und in vielen Bereichen eingesetztes Additiv und kann aufgrund seiner erfolgreichen Verwendung im Dentalbereich als Referenz für die Leistungsfähigkeit von 5 herangezogen werden.

### Beispiele

### Synthesen

### Herstellung von Di-(oxyethoxymethacrylsäure)-ethylendiamintetraessigsäureester 5

### Schritt 1: Darstellung von Ethylendiamintetraessigsäuredianhydrid 3.

29,23 g (10 mmol) käufliches EDTA werden in einem 250 ml Rundkolben mit 50,00 ml Acetanhydrid (529 mmol), 100 ml Dioxan sowie 10 ml Triethylamin vermischt. Diese Mischung wird unter Feuchtigkeitsausschluß 40 min bei 100°C gerührt. Nach dem Abkühlen wird der Feststoff der Suspension abfiltriert, mit Diethylether gewaschen, bis kein Triethylamin mehr nachweisbar ist und schließlich im Vakuum getrocknet.

Ausbeute: 24,1 g (94 %) weißes bis bräunliches Pulver

Identifikation:
Durch IR: keine Carbonsäurebande, Anhydridbande bei 1761,2 und 1808,7 Wellenzahlen [cm⁻¹]:
1808,7 (s, C=O), 1761,2 (s, C=O), 1466,2 (w), 1423,2 (w),1345,4 (w), 1251,7 (m), 1133,9 (m),1111,7 (m, part. overlap), 1070,8 (s), 1000,9 (m), 928,7 (s), 821,2 (m), 785,3 (w)

Durch GC (sil.): kein Peak bei 22,4 min. Das Anhydrid ist selbst nicht GC-gängig.

### Schritt 2: Umsetzung von EDTA-Dianhydrid 3 mit HEMA zu Verbindung 5

5,125 g (20 mmol) EDTA Dianhydrid werden mit 5,856 g (45 mmol) HEMA, 0,050 g BHT, 30 ml Dichlormethan und 1 ml Triethylamin versetzt und 1 h bei Raumtemperatur gerührt. Der Fortgang der Reaktion wird mittels GC überprüft.

Nach Abschluß der Reaktion wird die Mischung ungefähr zur Hälfte am Rotationsverdampfer eingeengt, in der Wärme mit ca. 20 ml Diethylether versetzt. Der entstehende Niederschlag wird abfiltriert, der Filterkuchen mit Diethylether gewaschen bis kein Amingeruch mehr feststellbar ist und die gesammelten Waschlösungen inclusive der Mutterlauge im Kühlschrank gelagert. Die Nachfällungen werden ebenfalls abfiltriert und mit Diethylether gewaschen.

Ausbeute: 5,808 g (56 %)
Verbindungstypische Daten:
Summenformel: C₂₂H₃₂N₂O₁₂
M = 516,50 g/mol
GC: Signal bei 30,397
¹H-NMR (ppm): 1,95 (s, 6H, CH₃), 3,08 (s, 4H, N-CH₂ CH₂), 3,67 (s, 4H, NCH₂CO), 3,81 (s, 4H, NCH₂CO), 4,33 (d, 8H, O-CH₂CH₂-O), 5,51 (s, 2H,CHCH₂), 6,07 (s, 2H,CH₃CCH₂), 10,09 (br., 2H, COOH)
¹³C-NMR (ppm): 18,21 (CH₃), 51,42 (NCH₂CH₂), 54,60 (NCH₂COOR), 56,00 (NCH₂COOH), 62,05 (CH₃CCOOCH₂), 62,94 (CH₂COOCH₂), 126,318 (CH₃CCH₂), 135,742 (CH₃C), 167,07 (CH₃CCOO), 169,50 (NCH₂COOR), 172,33 (COOH) TOF-MS (Probe silyliert): 660,2755 (3,3%, M^{+•}), 543,2393 (4,6%), 503,2243 (7,2%), 330,1241 (69,3%, ½ M^{+•}), 113,0666 (100%, C₅H₉O₂^{+•})
IR: 3416,9 (br., s, ν OH, ν CH₂, Olefin), 2960,3 (m, ν C-H, CH₂, CH₃), 2918,7 (m, ν C-H, CH₂, CH₃), 2850 (w, ν C-H, Methin), 1742,5 (s, ν C=O, Ester), 1717,8 (s, ν C=O, Säure), 1637,3 (s, ν C=C), 1454,3 (m, δ CH₂), 1405,1 (m), 1377,6 (m, δ CH₃), 1349,6 (m), 1321,8 (s), 1298,6 (s), 1201,6 (s), 1167,8 (s, ν C-O), 1096,7 (w)

### Haftversuche

### Adhäsivmischung - Grundlage G1

Als Harzgrundlage wurde die folgende, lichthärtende Zusammensetzung erstellt:

| | |
|---|---|
| 1. HEMA | 5,00 g |
| 2. TMPTMA | 2,50 g |
| 3. Bis-GMA | 2,50 g |
| 4. DABE | 0,090 g |
| 5. BHT | 0,020 g |
| 6. Campherchinon | 0,060 g |
| 7. Wasser | 2,50 g |
| 8. Aceton | 10,00 g |

Die Komponenten 1-8 werden unter Lichtschutz bei RT gerührt, bis eine klare, gelbliche Lösung G1 entstanden ist.

### Adhäsivmischung - Grundlage G2

Eine alternative, lichthärtende Mischung wurde aus den folgenden Komponenten erhalten:

| | |
|---|---|
| 1. HEMA | 10,00 |
| 2. UDMA | 5,00 |
| 3. Bis-GMA | 5,00 |
| 4. DABE | 1,80 |
| 5. BHT | 0,040 |
| 6. Campherchinon | 0,120 |
| 7. Wasser | 5,00 |
| 8. Aceton | 20,00 |

Die Komponenten 1-8 werden unter Lichtschutz bei RT gerührt, bis eine klare, gelbe Lösung G2 entstanden ist. Dieser Vorgang ist nach ca. 24 h abgeschlossen.

### Adhäsivmischung - Referenz, Adhäsiv R1

4,534 g (entspricht 2,00 g Basisharze) der Grundlage G1 werden mit 2,00 g (6,57 mmol) 4-META versetzt und bei RT bis zur vollständigen Auflösung des Feststoffes gerührt. Der Prozeß ist nach ca. 3,5 h abgeschlossen und ergibt eine 50%ige Lösung R1 bezüglich des Haftmonomergehaltes.

### Adhäsivmischung - Referenz, Adhäsiv R2

Eine Mischung aus 2,00 g (6,57 mmol) 4-META und 4,534 g Grundlage G2 werden bis zur Homogenität bei RT unter Lichtausschluß gerührt. Die klare, homogene Lösung R2 enthält 50% des Haftmonomers bezogen auf die Harzgehalte.

### Adhäsivmischung - Probe, Adhäsiv P1

Zu 4,534 g der Grundmischung G1 werden 1,173 g Verbindung 5 hinzugefügt und 20 h bei RT unter Lichtausschluß gerührt. Es entsteht eine leicht trübe, gelbe Lösung P1.

### Adhäsivmischung - Probe, Adhäsiv P2

Eine Mischung aus 4,544 g der Lösung G2 und 2,00 g Verbindung 5 wird ca. 20 min. bei RT unter Lichtausschluß gerührt. Es entsteht eine klare, gelbe Lösung P2.

### Adhäsiver gefüllter Dentalzement - Probe, Zement P3

Eine Mischung aus 4,649 g der Lösung G1 und 2,02 g Verbindung 5 wird ca. 20 min. bei RT unter Lichtausschluß gerührt. Zu der klaren, gelben Lösung werden Bariumaluminiumsilikatglaskeramiken portionsweise zu je 6,652 g, mittlere Partikelgröße 2 µm und 3,103 g einer feineren Aufmahlung mit einer mittleren Partikelgröße von 0,7 µm gemischt. Des Weiteren werden 0,350 g pyrogene Kieselsäure hinzugefügt und das Gemisch zu einer pastösen aber fließfähigen Masse homogenisiert.

### Adhäsiver gefüllter Dentalzement - Referenz, Zement R3

Eine Mischung aus 4,585 g von Lösung G1 und 2,01 g 4-META wird ca. 20 min. unter Lichtausschluß gerührt. Zu der klaren, gelben Lösung werden Bariumaluminiumsilikatglaskeramiken portionsweise zu je 6,638 g, mittlere Partikelgröße 10 µm und 3,110 g einer feineren Aufmahlung mit einer mittleren Partikelgröße von 2 µm gemischt. Des Weiteren werden 0,350 g pyrogene Kieselsäure hinzugefügt und das Gemisch zu einer pastösen aber fließfähigen Masse homogenisiert.

### Prüfung der Haftfestigkeit

Die Dentin- und Schmelzprobenkörper werden direkt vor ihrer Verwendung aus der isotonischen Kochsalzlösung entnommen, mit Wasser gespült und unverzüglich der Verarbeitung mit einem entsprechenden Bond zugeführt.
(I) Für das Referenzbond R1 und das Probe-Adhäsiv P1 wurde folgende Prozedur ausgewählt:
   Um eine frische Präparation zu simulieren werden die freiliegenden Dentin- und Schmelzflächen auf feinem Schmirgelpapier (1000er Körnung) feucht beschliffen. Die resultierende Fläche soll plan sein und nicht austrocknen. Im Notfall muss nachträglich mit Wasser benetzt werden. Direkt vor der Applikation wird überschüssige Feuchtigkeit verblasen und R1 bzw P1 mittels eines Schwämmchens oder eines Pinsels 2-3 Mal aufgetragen und 30 s einwirken gelassen. Es soll nach dem Verblasen des Lösungsmittels eine geschlossene Adhäsivschicht auf der Zahnhartsubstanz verblieben sein. Das Adhäsiv wird dann 20 s mit einer geeigneten Blaulichtquelle lichtgehärtet. Danach wird auf der gehärteten Fläche ein Silikonring mit einem Innendurchmesser von ca. 5 mm aufgesetzt ohne die bestehende Inhibitionsschicht des Adhäsivs zu verwischen. In die Öffnung des Silikonringes wird dann ein geeignetes lichthärtendes Füllungskomposit auf Methacrylatbasis aufgetragen und 20 s lichtgehärtet. Die fertigen Proben werden h im 37°C Probenschrank bei 100% rF gelagert und nach 24h die Scherhaftung bestimmt.
(II) Für die, aus der alternativen Adhäsivgrundlage hergestellten, Referenz- und Prüfadhäsive R2 und P2 werden 2-3 Mal aufgetragen und mit dem Applikationsinstrument ca. 20 s einmassiert. Dann wird 10 s lichtgehärtet, ein geeignetes Komposit mit Hilfe eines Silikonringes aufgetragen, nochmals 40 s lichtgehärtet und 24 h im 37°C Probenschrank bei 100% rF gelagert. Die erhaltenen Haftwerte sind Mittelwerte aus mindestens 5 Messungen in [N/mm²].
(III) Für den Haftzement wird die Kavität wie unter (I) beschrieben vorbereitet. Der Zement wird dann mittels eines Schwämmchens oder Pinsels auf die angeschliffene Fläche in geschlossener Schicht aufgetragen und 20 s in das Probendentin eingearbeitet. Dann wird der Zement mit dem Luftbläser gleichmäßig verteilt und 20 s lichtgehärtet. Hierbei ist darauf zu achten, dass sich keine Ansammlungen bilden ("Pooling") und die Oberfläche des Probenkörpers gleichmäßig benetzt ist. Nach der Härtung wird ein Silikonring mit einem Innendurchmesser von 5 mm aufgesetzt ohne die Inhibitionsschicht innerhalb des Ringes zu beschädigen und ein Füllungskomposit eingebracht. Nach der 40 s Lichthärtung wird die Probe im 37°C Wasserdampfbad für 24 h gelagert und dann die Scherhaftung ermittelt. Die angegebenen Haftwerte sind Mittelwerte aus mindestens 5 Einzelmessungen.

### Haftprüfungen auf Humandentin:

| | Haftwerte |
|---|---|
| R1 | 25,6 [N/mm²] |
| P1 | 29,4 [N/mm²] |
| R2 | 20,8 [N/mm²] |
| P2 | 21,5 [N/mm²] |
| R3 | 17,6 [N/mm²] |
| P3 | 22,3 [N/mm²] |

Geräte:

| | |
|---|---|
| GC: | Shimadzu, Trägergas Stickstoff, 1ml/min, Säule HP5, 30 m 50 °C/10°C min⁻¹/300°C/25 min |
| ¹H-, ¹³C-NMR: | Varian 600 MHz, Unity Plus Inova 500 MHz Probenlösung in CDCl₃ |
| MS: | ESI MS: Waters-Micromass, Quattro LCZ Micro-TOF: Bruker, Daltoniks, Bremen Probenlösung in MeOH (0,01 mg in 2 ml) |
| IR: | Vector 22, KBr, 10 Scans |

## Patentansprüche

1. Adhäsive Zusammensetzung, die eine organische, polymerisierbare Matrix enthält, welche
(a) als haftvermittelnde Komponente ein Derivat einer Alkylendiamin-N,N,N,N-tetraessigsäure der Formel 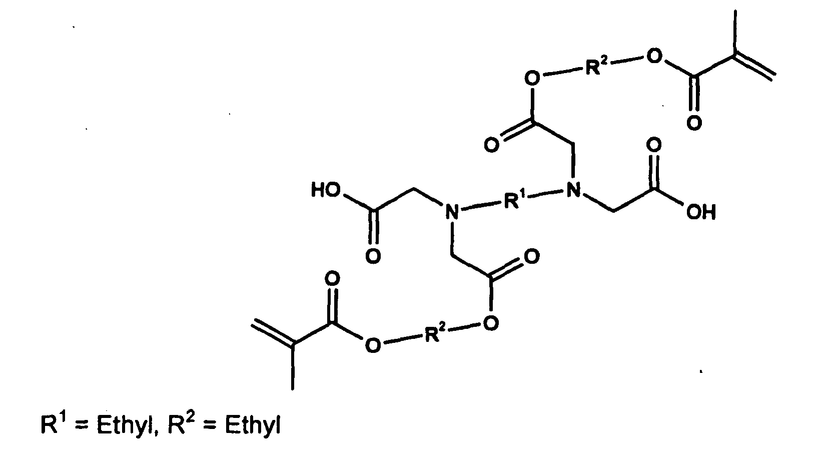
(b) geeignete Polymerisationinitiatoren und/oder Polymerisationskatalysatoren
(c) geeignete organische Lösungsmittel und/oder Wasser enthält.

2. Zusammensetzung nach Anspruch 1, wobei die Mischung zur Verbesserung der physikalischen Eigenschaften feinkörnige mikro- und/oder nanoskalige mineralische Zusätze aus der Gruppe der Quarze, Kieselsäuren, Aerosile, Apatite, Glaskeramiken, Fluoro-, Boro- und Alumosilikate, schwerlösliche Barium- oder Strontiumverbindungen, speziell Bariumsulfat, Eisenoxide, Zirkon-, Aluminiumoxid und/oder feste, schwerlösliche organische Polymere, beispielsweise Polymethylmethacrylate, Polymethylacrylate, - styrole, -ethylene, -carbonate, -urethane, -ester, -amide und Silikone enthält.

3. Zusammensetzung nach Anspruch 1, worin die organische polymerisierbare Matrix acrylat- oder methacrylatbasierende Monomere und/oder radikalisch härtbare Präpolymere enthält.

4. Verwendung einer Zusammensetzung nach Anspruch 2 oder nach Anspruch 3, wobei das Präparat in der Zahnheilkunde, insbesondere in adhäsiven Bondingmaterialien, Zementen oder Füllungswerkstoffen, eingesetzt wird.

## Claims

1. Adhesive composition comprising an organic polymerizable matrix comprising
(a) adhesion-promoting component comprising a derivative of an alkylenediamine-N,N,N,N-tetraacetic acid of the formula 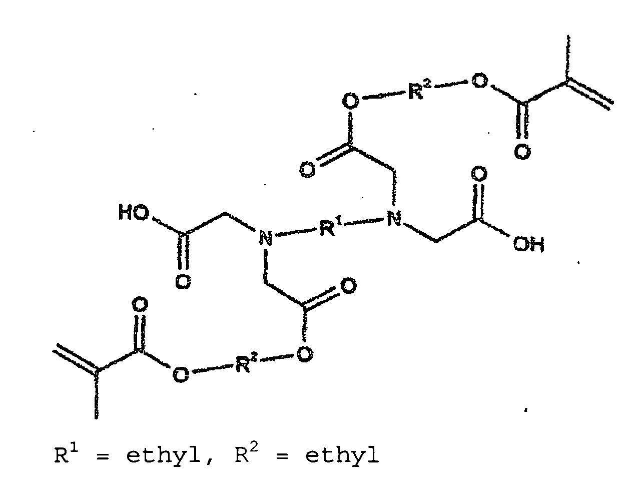
(b) suitable polymerization initiators and/or polymerization catalysts
(c) suitable organic solvents and/or water.

2. Composition according to Claim 1, comprising for improved physical properties finely granular micro- and/or nanoscale mineral additions for the group consisting of quartzes, silicas, aerosols, apatites, glass ceramics, fluoro-, boro- and aluminosilicates, sparingly soluble barium or strontium compounds, specifically barium sulphate, iron oxides, zirconium oxide, aluminium oxide and/or solid, sparingly soluble organic polymers, for example poly(methyl methacrylate)s, poly(methyl acrylate)s, polystyrenes, polyethylenes, polycarbonates, polyurethanes, polyesters, polyamides and silicones.

3. Composition according to Claim 1, wherein the organic polymerizable matrix comprises acrylate- or methacrylate-based monomers and/or free-radically curable prepolymers.

4. Use of a composition according to Claim 2 or according to Claim 3 in dentistry, more particularly in adhesive-type bonding materials, cements or filling materials.

## Revendications

1. Composition adhésive, qui contient une matrice organique polymérisable contenant
(a) en tant que composant adhésif, un dérivé d'un acide alkylène-diamine-N,N,N,N-tétraacétique de formule 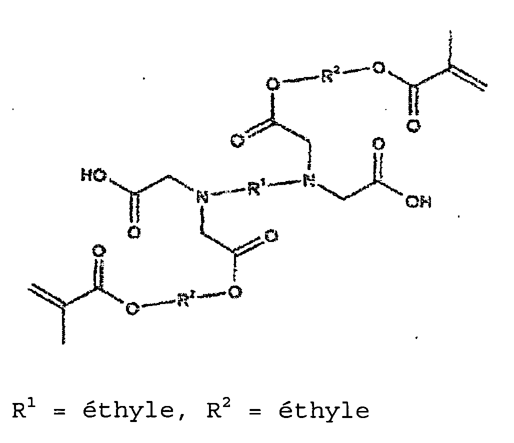
(b) des initiateurs de polymérisation et/ou des catalyseurs de polymérisation appropriés,
(c) des solvants organiques appropriés et/ou de l'eau.

2. Composition selon la revendication 1, dans laquelle le mélange contient, pour améliorer les propriétés physiques, des additifs minéraux micro- et/ou nanométriques à grains fins du groupe constitué par les quartz, les silices, les aérosils, les apatites, les vitrocéramiques, les fluoro-, boro- et alumosilicates, les composés de baryum ou de strontium difficilement solubles, notamment le sulfate de baryum, les oxydes de fer, l'oxyde de zirconium, d'aluminium et/ou les polymères organiques solides difficilement solubles, par exemple les polyméthacrylates de méthyle, les polyacrylates de méthyle, les polystyrènes, les polyéthylènes, les polycarbonates, les polyuréthanes, les polyesters, les polyamides, et les silicones.

3. Composition selon la revendication 1, dans laquelle la matrice organique polymérisable contient des monomères à base d'acrylate ou de méthacrylate et/ou des prépolymères durcissables par voie radicalaire.

4. Utilisation d'une composition selon la revendication 2 ou selon la revendication 3, dans laquelle la préparation est utilisée en dentisterie, notamment dans des matériaux de liaison adhésifs, dans des ciments ou dans des matériaux de remplissage.
